(19) Europäisches Patentamt
European Patent Office
Office européen des brevets

(11) **EP 0 588 621 B1**

(12) **EUROPEAN PATENT SPECIFICATION**

(45) Date of publication and mention
of the grant of the patent:
**15.12.1999 Bulletin 1999/50**

(51) Int. Cl.⁶: **A61K 35/78**

(21) Application number: **93307278.7**

(22) Date of filing: **15.09.1993**

(54) **Neutral lipids from endosperm of job's tears and compositions including them**

Neutrale Lipide aus dem Endosperm von Coix lacryma jobi und sie enthaltende Zusammensetzungen

Lipides neutres tirés de l'endosperme de Coix lacrima jobi et compositions les contenant

(84) Designated Contracting States:
**DE DK FR GB SE**

(30) Priority: **16.09.1992 CN 92110677**
**17.09.1992 CN 92110839**
**01.01.1993 CN 93100735**

(43) Date of publication of application:
**23.03.1994 Bulletin 1994/12**

(73) Proprietors:
• **Li, Da Peng**
**Hangzhou (CN)**
• **ZHEJIANG PROVINCIAL HOSPITAL OF TRADITIONAL CHINESE MEDICINE**
**Hangzhou (CN)**

(72) Inventor: **Li, Da Peng**
**Hangzhou (CN)**

(74) Representative:
**Harrison, David Christopher et al**
**MEWBURN ELLIS**
**York House**
**23 Kingsway**
**London WC2B 6HP (GB)**

(56) References cited:
**FR-M- 4 618**

• **CHEMICAL ABSTRACTS, vol. 113, no. 22, 26 November 1990, Columbus, Ohio, US; abstract no. 197985f, & CN-A-1 034 859 (XU, WANGANG) 23 August 1989**
• **DATABASE WPI Week 8404, Derwent Publications Ltd., London, GB; AN 84-021535 & JP-A-58 213 719 (Y. ARAKI) 12 December 1983**
• **CHEMICAL ABSTRACTS, vol. 99, no. 11, 12 September 1983, Columbus, Ohio, US; abstract no. 85114g, H. YOSHIDA ET AL 'Lipids of Coix ma-yuen (pearl-barley)'**
• **DATABASE WPI Week 7806, Derwent Publications Ltd., London, GB; AN 78-11580A & JP-A-52 156 936 (Y. OGAWA) 27 December 1977**

## Description

The invention concerns neutral lipids from the endosperm of Job's tears (Coix lachryma-jobi), a method to extract and refine the lipids from the endosperm and an anti-tumor composition including the neutral lipids, for injection or oral administration.

The dry endosperm of Job's tears has been used as a traditional medicine and nourishment for thousands of years in China.

In 1961, Tyunosin Ukita and Akio Tanimura (Ukita T et al., Chem. Pharm. Dull, 9(1):43,1961) reported an extraction of coixenolide as an anti-tumor element from the endosperm of Job's tears and the results of this anti-tumor activity tests. They reported also the formula of coixenolide as $C_{38}H_{70}O_4$; however, the pharmaceutical preparation thereof was not mentioned. It was reported to inhibit the growth of mouse Ehrlich ascites tumor. Coixenolide was extracted by the following steps:

Powder of the endosperm extracted by acetone 3 times - extract - dissolved in petrol ether, filtered and concentrated - brown syrup - dissolved in petrol ether, run through silica gel column by petrol ether - eluate - add 0.2N KOH to separate acidic element - Neutral oil - run through alumina column and silica column - coixenolide.

Coixenolide was obtained as a pure compound and exhibited strong anti-tumor activity. But there is no report about its clinical application. Because of the two kinds of column chromatography (alumina and silica) used in its extraction and refinement the yield was quite low, thus it was very expensive to obtain and no commercial products have resulted.

In the eighties, Si Pei-hai obtained crude oil from endosperm of Job's tears directly with petrol ether and made it up as an intravenous emulsion (Si Pei-hai, The Extraction of the Oil of the Endosperm of Job's Tears and the Preparation of its Emulsion, Zhejiang Pharmacology, 3(6):18-20, 1986). The extraction and refinement steps of the oil of the endosperm of Job's tears was as follows:

Powder of the endosperm of Job's tears - extracted by petrol ether (3 times) - extract - absorption, decoloration and evaporation - oil of the endosperm of Job's tears.

The oil had a relative density of 0.9033-0.9057 (20°C), diopter 1.4670-1.4708 (20°C), iodine value 83-90 and acid value <36. The intravenous emulsion comprised the oil extracted with above method (as main component), and two emulsifiers; Spans* and Tween*. The composition was:

| Oil from the endosperm of Job's tears | 10 g |
|---|---|
| Spans*-80 | 1 g |
| Tween*-80 | 1.5 g |
| Water for injection use | q.s to 100 ml |

*Trade Marks

The intravenous emulsion was only used for research into pharmaceutical preparations and its distribution in the body.

There are following obstacles against the intravenous emulsion being used for human clinical use: *Trade Marks

1. The main component, the oil from the endosperm of Job's tears, was not pure and safe enough to meet the requirement of Chinese Pharmacopoeia, e.g. its acid value was controlled just below 36.
2. Tween and Spans can get through cell membrane, are lysoactive and toxic. Only if suitable isoosmoticum is used can lysogenesis be prevented. Most commercial intravenous emulsion products in England, Germany, United States, France, China and Japan do not use emulsifiers such as Tween and Spans.
3. The above composition does not include the essential element of isoosmoticum, thus lacks utility.

The aim of the present invention is to provide an extraction and refinement for obtaining neutral lipids from the endosperm of Job's tears (hereinafter NLEJ). The method has simple steps and low expense, but yields a standard of neutral lipids meeting the need of intravenous use in anti-tumor treatments.

Another aim of the present invention is to provide a pharmaceutical composition with NLEJ, as for example an O/W emulsion as anti-tumor drug. The composition is as safe and reasonable as to be capable of oral, intravenous or intraartery administration.

Brief Description of Particular Embodiments

NLEJ concerned in the present invention is a light yellow transparent liquid at room temperature. NLEJ is easily dissolved in petrol ether, ether and benzol, able to be dissolved in acetone and dissolved a little in methanol, alcohol, and cannot be dissolved in water. Tested as an oil, NLEJ has physicochemical constants as follows: acid value <0.20, iodine value 95.00-107.00, saponification value 185.00-195.00, relative density 0.915-0.918 (20°C), diopter 1.470-1.475 (20°C). NLEJ comprise triglyceride (91.48 ± 3.43%), diglyceride (1.47 ± 0.63%), monoglyceride (5.75 ± 3.19%) and akylacylacetin (1.0 ± 0.78%). The lipoclastic fatty acid residues comprise hexadecanoic, octodecanoic, octadecenoic and octadecadienoic acid.

One specific method for the extraction and refinement of NLEJ is as follows:

Extraction by organic solvent: the organic solvent is e.g. acetone, petrol ether, ether, alcohol or hexane. The extraction method is e.g. percolation, filtering or seepage through a powder of endosperm of Job's tears. Crude oil will be extracted with large amount of foreign matter, e.g. free fatty acids, pigments;
Absorption and decoloration: common absorbent is used i.e. 1% active carbon, 3% white potters clay, 10% alumina, or others. Yellow oil will be obtained after the crude extract is absorbed;
Alkalizing saponification: after a suitable amount of alkaline solution (e.g. NaOH or KOH) is added for saponification, an emulsion will be the result;
Deemulsification by acetone: a suitable amount of acetone will turn the emulsion transparent;
Liquid-liquid extraction: a suitable amount of second organic solvent, e.g. petrol ether, ether or hexane; then extract and settle, discard the phase containing mainly acetone (containing acidic element, water), evaporate the organic solvent. The product is a residue, mainly NLEJ of the present invention.
Refinement: between alkalization saponification and deemulsification with acetone, a step of washing the emulsion with hot water is added; and/or after liquid-liquid extraction, one step of decoloration and/or washing by hot water is added if necessary.

Then, NLEF of high purity meeting the need of intravenous use is obtained.

A typical emulsion of the present invention has pharmaceutical standard NLEJ as main component, soy (or egg) lecithin as emulsifier, and enough water. It also contains glycerol, sorbitolum or the like as isoosmoticum. It could also contain other conventional anti-tumor drugs. The method of emulsification includes two conventional steps, homogenization and dispersion. Within the pharmaceutical composition the content of NLEJ could vary in wide range. It is preferably an O/W type emulsion. Within 100 ml of the emulsion, NLEJ content may range from 5 to 25 g.

NLEJ and its pharmaceutical composition of the present invention has a high anti-tumor activity. The latter can be especially used in the therapeutics of lung cancer, carcinoma, hepatic and other metastasizing cancer in middle or late stage. It can enhance body immunity and protect body from many diseases, thus can also be used in treatment of the immune deficiency diseases. Combination of NLEJ emulsion with low dose chemotherapy can strengthen the anti-tumor activity and reduce the toxicity of chemotherapy.

Testings of anti-tumor activity of NLEJ and its pharmaceutical composition on human tumor are seen below in Example 9 and Example 10. The effects of this pharmaceutical composition on immunity, the enhancement of the combination with low dose chemotherapy and the protective effect on the decrease of leucocyte induced by chemotherapy will be illustrated respectively in Example 11, Example 12 and Example 13, and the clincal effects of this pharmaceutical composition are described in Example 14.

The compounds of this invention were administered at a therapeutically effective dosage, i.e. the amount which, when administered to a mammal in need thereof, is therapeutically sufficient for effective treatment on tumor diseases. Administration of the active compounds herein could be intravenous, intraarterial or oral.

In the accompanying drawings:

Fig 1 shows the effect of NLEJ on Proliferation of Leukaemia P388, L1210;
Fig 2 shows the effect of NLEJ on Formation of Cell Colony of Colon Tumour M 7609;
Fig 3 shows the therapeutic effect of NLEJ Emulsion on Metastasis of Melanoma (B16) in Lung in vivo (every third day);
Fig 4 shows the therapeutic effect of NLEJ Emulsion on Metastasis of Melanoma (B16) in Lung in vivo (iv, every day); and
Fig 5 shows the therapeutic effect of NLEJ Emulsion on Metastasis of Melanoma (B16) in Lung in vivo (ip, every day).

The following Examples are given to enable those skilled in the art to more clearly understand and to practice the present invention. They should not be considered as limiting the scope of the invention, but merely as being illustrative and representative thereof.

**Example 1**

Extraction and Refinement of NLEJ

100 kg powder of the endosperm of Job's tears was extracted with acetone, 5kg extract was obtained after the evaporation of acetone, absorbed and decoloured, 152 g NaOH (or relative amount of KOH) dissolved as 5% hot alkaline solution was added for alkalizing saponification, weight of alkaline added depended on the acid value of extract, high temperature of alkaline made the saponification more rapid and thorough, separated the emulsion after settled, washed the emulsion 2-3 times until the emulsion turned to be neutral (pH 6-7), 1:1 (v/v) acetone was added for deemulsification, after settlement extract the acetone phase with enough petrol ether, settled and discarded acetone, NLEJ was remained in petrol ether , absorbent was added for pigments and pyrogen, neutral oil was obtained after filtration and petrol ether evaporation, then boiled for 0.5 hr with hot distilled water under vacuum, settled and heated the neutral oil to 100°C to evaporate water, the absorbent was added again and filtered out, 2kg light yellow NLEJ concerned in present invention was obtained, packaged it for preparing intravenous and oral emulsion after heating to 160°C for 2 hr for sterilization. The absorbent mentioned above is active carbon, white pottery clay alumina or others.

**Example 2-8**

Compositions of NLEJ emulsion

The compositions of oral use, intravenous and intraartery use will be illustrated in detail in Example 2-8.

**Example 2**

| NLEJ | 10.0 g |
|---|---|
| Soy lecithin for injection use | 1.5 g |
| Glycerol for injection use | 2.5 g |
| Water for injection use | q.s. to 100 ml |

The emulsion was oral, intravenous or intraartery emulsion.

**Example 3**

| NLEJ | 10.0 g |
|---|---|
| Lecithin for injection use | 1.2 g |
| Glycerol for injection use | 2.5 g |
| Water for injection use | q.s. to 100 ml |

The emulsion was intravenous or intraartery emulsion.

**Example 4**

| NLEJ | 15.0 g |
|---|---|
| Pluronic F88 | 2.0 g |
| Glycerol | 2.5 g |
| Distilled water | q.s. to 100 ml |

The emulsion was oral emulsion.

**Example 5**

| NLEJ | 15.0 g |
|---|---|
| Soy lecithin for injection use | 2.0 g |
| Glycerol for injection use | 2.5 g |
| Water for injection use | q.s. to 100 ml |

The emulsion was intravenous or intraartery emulsion.

**Example 6**

| NLEJ | 5.0 g |
|---|---|
| Phospholipid | 0.75 g |
| Glycerol | 1.25 g |
| Distilled water | q.s. to 100 ml |

The emulsion was oral emulsion.

**Example 7**

| NLEJ | 2.5 g |
|---|---|
| Phospholipid | 2.5 g |
| Glycerol | 3.0 g |
| Distilled water | q.s. to 100 ml |

The emulsion was oral emulsion.

**Example 8**

| NLEJ | 1000 g |
|---|---|
| Soy lecithin for injection use | 120 g |
| Glycerol for injection use | 250 g |
| Water for injection use | q.s. to 10000 ml |

The emulsion was intravenous or intraartery emulsion.

**Example 9**

Anti-tumor Activity of NLEJ in vitro

(1) Effect of NLEJ on Inhibition of Proliferation Leukaemia P388 and L1210 Cultured Cells

Methods

20 ul NLEJ was added in deferent concentration into the 96 wells microplate of cultured leukaemia cell lines in duplicate, 20 ul pbs was added into the wells of control, $9 \times 10^5$ cells was in each well, the highest concentration of NLEJ was 100 ul/ml, cells was incubated for 48 hr at 37°C and under 5% $CO_2$, cells were counted with Coulter Counter, the inhibition ratio and the concentration of drug which inhibited 50% of the colony proliferation of the tumor cells ($IC_{50}$) was calculated,
[0038]   Inhibition ratio= ((cell number of control- cell number of treatment)/(cell number of control- cell number at the beginning of test))x 100%

Results

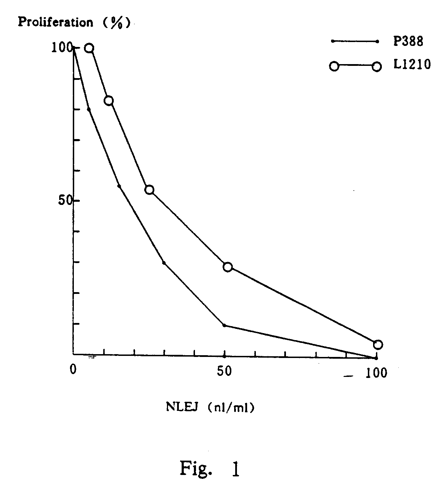

NLEJ showed evident anti-tumor activity in leukaemia P388 and L1210 cells in vitro. The dosage above 50 ul/ml inhibited almost all cell's proliferation. The $IC_{50}$ of NLEJ on P388 and L1210 cells were 15.2 ul/ml and 28.8 ul/ml respectively. Their coefficients of correlation were 0.9136 and 0.9454 respectively(Fig. 1 and Table 1).

Table 1

| Effect of NLEJ on Proliferation Leukaemia P388, L1210 and Colon Tumor M7609 Cells $IC_{50}$ of NLEJ (ul/ml) | | |
|---|---|---|
| P388 | L1210 | M7609 |
| 15.1 | 28.8 | 11.9 |

(2) Test on Formation Inhibition of Colon Tumor M7609 Cell Colony

Methods

0.1 ml suspension of colon tumor(M7609) cells (2000/ml) at logarithmic stage prepared after digested by 0.15% trypsin was added in 3.8 ml RPMI 1640 medium containing 20% bovine serum in d-4 cm round cell container for incubation, 0.1 ml NLEJ of different concentration was added in the container after 24 hr incubation at 37°C and under 5% $CO_2$, the cells incubated 9 days more, after dyeing with 0.1% crystal violet in alcohol the colony was counted, colony formation inhibition ratio and $IC_{50}$ was calculated.

Results

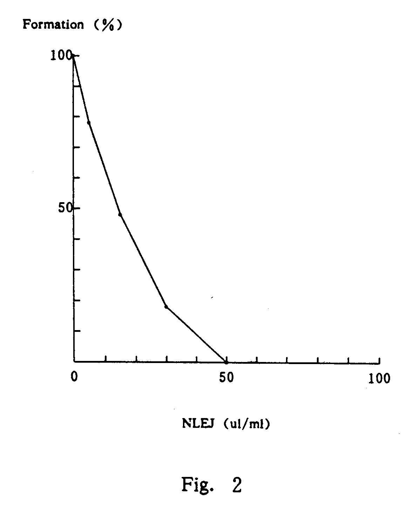

NLEJ markedly inhibited the formation of cell colony of colon tumor M7609. The concentration of 50 ul/ml inhibited all the formation. The $IC_{50}$ of NLEJ on the formation of cell colony of colon tumor M7609 was 11.88 ul/ml. Their coefficient of correlation was 0.9445 (Fig. 2 and Table 1).

**Example 10**

Therapeutic Effect of NLEJ Emulsion on Metastasizing Tumor in vivo

(1) Therapeutic Effect of NLEJ Emulsion on Growth of Human Hepatoma (QGY) in vivo

Methods

Hepatoma (QGY) tissues growing luxuriantly were homogenized in physical saline (1:4) and prepared as suspension, each nude mouse was inoculated subcutaneously 0.2 ml of the suspension at its armpit, nude miee were divided into groups at random the next day of inoculating, the doses of NLEJ emulsion were 25 ml/kg, 12.5ml/kg and 6.25 ml/kg, the emulsion administrated intravenously at the tail of nude mouse every day within 10 days, one group of mice were administered with cyclophosphamide (CTX) taking a dose of 30 mg/kg intraperitoneal every day within 7 days. the nude mice of control group were administered with relative emulsion just absent NLEJ (blank emulsion) taking a dose of 25 ml/kg intravenously at the tail of nude mouse every day within 10 days. Tumor tissues of all nude mice were sampled and weighted 30 days after inoculating.

Results

All doses of NLEJ emulsion, 25 ml/kg, 12.5 ml/kg and 6.25 ml/kg, showed good therapeutic effect on the growth of human hepatoma (QGY), and good dose-activity correlation relationship, the highest dose (25 ml/kg) got inhibition ratio as almost high as the treatment of CTX (Table 2).

Table 2

| Therapeutic Effect of NLEJ Emulsion on Growth of Human Hepatoma (QGY) in vivo | | | | | |
|---|---|---|---|---|---|
| Sample | Dose | Number | Tumor (g) | inhibition (%) | P value |
| NLEJ | 6.25ml/kg | 5/5 | 1.68 ± 0.52 | 49.70 | <0.01 |

Table 2 (continued)

| Therapeutic Effect of NLEJ Emulsion on Growth of Human Hepatoma (QGY) in vivo | | | | | |
|---|---|---|---|---|---|
| Sample | Dose | Number | Tumor (g) | inhibition (%) | P value |
| NLEJ | 12.5 ml/kg | 5/5 | $1.02 \pm 0.31$ | 61.08 | <0.01 |
| NLEJ | 25.0 ml/kg | 5/5 | $0.54 \pm 0.21$ | 83.83 | <0.01 |
| CTX | 30.0 mg/kg | 5/5 | $0.28 \pm 0.08$ | 91.00 | <0.01 |
| Control | 25.0 ml/kg | 5/5 | $3.34 \pm 0.86$ | - | - |

(2) Therapeutic Effect of NLEJ Emulsion on Growth of Human Small Cell Lung Cancer (SPC) in vivo

Methods

SPC tumor tissues growing luxuriantly were cut into pieces of 2 mm, one of the pieces were inoculated under the skin of armpit of each nude mouse, nude mice were divided into groups at random the next day of inoculating and administered, the dose of NLEJ emulsion was 25 ml/kg iv x10 (qd), one group of mice were administered with dacarbazine (DTIC) taking a dose of 40 mg/kg iv x 10 (qd), the nude mice of control group were administered with blank emulsion taking a dose of 25 ml/kg iv x 10 (qd). tumor tissues of all nude mice were sampled and weighted 30 days after inoculating.

Results

The dose of 25 ml/kg of NLEJ showed good therapeutic effect on human small cell lung cancer. the inhibition ratio was as high as 62.35% (Table 3).

Table 3

| Therapeutic Effect of NLEJ Emulsion on Growth of Human Small Cell Lung Cancer (SPC) in vivo | | | | | |
|---|---|---|---|---|---|
| Sample | Dose | Number | Tumor (g) | inhibition (%) | P value |
| NLEJ | 25 ml/kg | 5/5 | $1.34 \pm 0.32$ | 62.35 | <0.01 |
| DTIC | 40 mg/kg | 5/5 | $0.18 \pm 0.04$ | 94.94 | <0.01 |
| Control | 25 ml/kg | 5/5 | $3.56 \pm 0.83$ | - | - |

(3) Therapeutic Effect of NLEJ Emulsion on Metastasis of Melanoma (B16) in Lung in vivo

Methods

Melanoma B16 tissues growing at logarithmic stage were digested with 0.15% trypsin, 0.2 ml suspension of $2.5 \times 10^5$ cells/ml in RPMI 1640 medium from the tissues inoculated intravenously into the tail of each C57BL/6 mouse, mice were divided into groups at random 3 day after inoculating and administered differently, mice were killed the 21 days after inoculating, metastasized colonies of melanoma in mouse's lung were counted to calculate the inhibition ratio and compare the different effects of different administrations of NLEJ emulsion on metastasis of melanoma in lung, 100mg/kg cyclophosphamide (CTX) was administered in the same way to compare with NLEJ emulsion.

Results

The different effect of different administrations on metastasis of melanoma

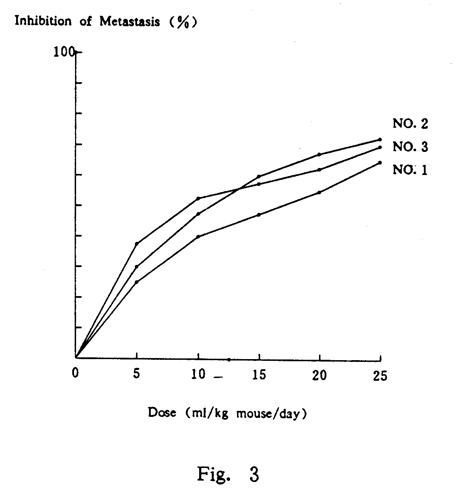

(i) All the combination of the dose (6.25ml/kg, 12.5ml/kg and 25ml/kg) and the administration (every third day, 4 times and intravenously at tail) of NIEJ emulsion showed evident inhibition effect on metastasis of melanoma B16 (Table 4, Fig. 3).

Table 4

| Therapeutic Effect of NLEJ Emulsion on Metastasis of Melanoma (B16) in Lung in vivo (every third day) | | | | | | |
|---|---|---|---|---|---|---|
| Sample | Dose (ml/kg) | Administration | Number | Colony No. ($\overline{X}\pm SD$) | Inhibition (%) | P value |
| NLEJ | 6.25 | iv $\times$ 4 (3,5,7,9) | 30/30 | 93.43$\pm$15.74 | 36.88 | <0.01 |
| NLEJ | 12.5 | iv $\times$ 4 (3,5,7,9) | 30/30 | 79.87$\pm$19.02 | 46.08 | <0.01 |
| NLEJ | 25.0 | iv $\times$ 4 (3,5,7,9) | 30/30 | 47.83$\pm$15.53 | 67.76 | <0.01 |
| CTX | 100mg/kg | ip $\times$ 2 (3,5) | 30/30 | 18.80$\pm$ 8.50 | 95.90 | <0.01 |
| CK | 25.0 | iv $\times$ 4 (3,5,7,9) | 30/30 | >147 | - | - |

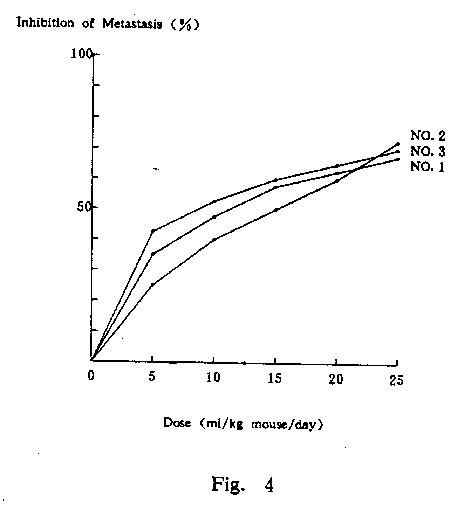

(ii) All the combination of the dose (6.25ml/kg, 12.5ml/kg and 25/kg) and the administration (every day, 7 times and intravenously at tail) of NLEJ emulsion showed evident inhibition effect on metastasis of melanoma B16, too (Table 5, Fig. 4).

Table 5

| Therapeutic Effect of NLEJ Emulsion on Metastasis of Melanoma (B16) in Lung in vivo (iv, every day) | | | | | | |
|---|---|---|---|---|---|---|
| Sample | Dose (ml/kg) | Administration | Number | Colony No. ($\overline{X}\pm SD$) | Inhibition (%) | P value |
| NLEJ | 6.25 | iv $\times$ 7 | 30/30 | 83.23$\pm$22.82 | 44.87 | <0.01 |
| NLEJ | 12.5 | iv $\times$ 7 | 30/30 | 51.87$\pm$13.28 | 63.31 | <0.01 |
| NLEJ | 25.0 | iv $\times$ 7 | 30/30 | 32.83$\pm$11.80 | 78.77 | <0.01 |
| CTX | 100mg/kg | ip $\times$ 2 (3,5) | 30/30 | 8.0 $\pm$ 6.22 | 98.67 | <0.01 |
| CK | 25.0 | iv $\times$ 7 | 30/30 | >155 | - | - |

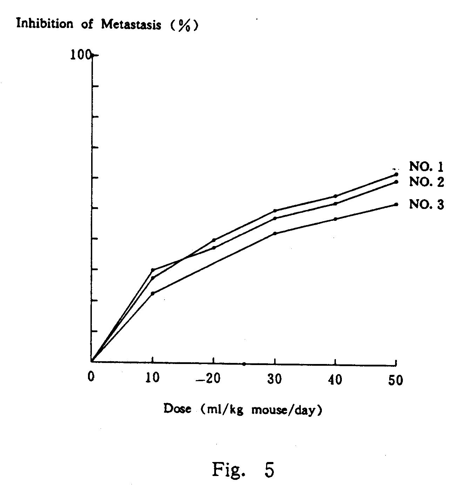

(iii) All the combination of the dose (6.25ml/kg, 12.5/kg and 25/kg) and the administration (every day, 7 times, and intraperitoneal) of NLEJ emulsion showed also evident inhibition effect on metastasis of melanoma B16 (Table 6, Fig.5).

Table 6

| Therapeutic Effect of NLEJ Emulsion on Metastasis of Melanoma (B16) in Lung in vivo (ip, every day) | | | | | | |
|---|---|---|---|---|---|---|
| Sample | Dose (ml/kg) | Administration | Number | Colony No. ($\overline{X}\pm SD$) | Inhibition (%) | P value |
| NLEJ | 6.25 | ip $\times$ 7 | 30/30 | 80.47$\pm$18.18 | 29.25 | <0.01 |
| NLEJ | 12.5 | ip $\times$ 7 | 30/30 | 68.50$\pm$16.77 | 39.64 | <0.01 |
| NLEJ | 25.0 | ip $\times$ 7 | 30/30 | 47.07$\pm$13.72 | 58.28 | <0.01 |
| CTX | 100mg/kg | ip $\times$ 2 (3,5) | 30/30 | 0 | 100 | <0.01 |
| CK | 25.0 | ip $\times$ 7 | 30/30 | >114 | - | - |

**Example 11**

Enhancement of Mouse Immunity from NLEJ Emulsion

(1) Inducement of Proliferation of Mouse Spleen Lymphatic Cell from NLEJ Emulsion in vitro

Methods

Spleen cells were obtained aseptically from C57BL/6 mouse, cell density was regulated to be $1 \times 10^7$/ml with RPMI-1640 medium, each cell hole contained 100ul medium, 100ul drugs and 50ul ConA, here mentioned drugs were NLEJ emulsion (4 concentrations), lentinanin (4 concentrations), blank emulsion and water, 4 duplications for each treatment, cells were incubated for 48 hr at 37°C and under 5% $CO_2$, then $^3$H-Tdr was added to the wells (0.5uci/well), incubated 20 hr more, cells were collected then and CPM value were measured with liquid scintillation counter.

Results

It can be seen in Table 7 that the cells incubated with both NLEJ and lentinanin got higher CPM value, i.e. both NLEJ and lentinanin induced the proliferation of mouse spleen lymphatic cell in vitro. The correlation relationship between dose and CPM value was also showed.

Table 7

| Inducement of Proliferation of Mouse Spleen Lymphatic Cell from NLEJ Emulsion and Lentinanin in vitro | | |
|---|---|---|
| Treatment | Sample (Concentration) | CPM ($\overline{X}\pm$SD) |
| NLEJ | 100(1:4) | 7765 ± 1008** |
| NLEJ | 100(1:2) | 7165 ± 1163** |
| NLEJ | 100(1:1) | 6540 ± 946** |
| NLEJ | 100(mother) | 6230 ± 1130** |
| Lentinanin | 100(1:4) | 11555 ± 1279** |
| Leutinanin | 100(1:2) | 11025 ± 1133** |
| Leutinanin | 100(1:1) | 7700 ± 747** |
| Leutinanin | 100(mother) | 7420 ± 957** |
| Blank | 100(mother) | 4612 ± 719 |
| Control | 100(mother) | 4795 ± 487 |

** P<0.01 compared with Blank and Control

(2) Influence of NLEJ Emulsion on the Activity of Natural Killer Cell of Mouse in vitro

Methods

Spleen cells were obtained aseptically from C57BL/6 mouse as effective cells, cell density was regulated to be $1 \times 10^6$/ml, the density of aim cells, YAC-1 cells, were regulated to be $1 \times 10^4$, the two suspensions of cells were mixed in 1:1 (v/v), i.e. the ratio between effective and aim cell was 1:100, $^3$H-Tdr were added to the wells (0.5uci/well), cells were incubated for 48hr at 37°C and under 5% $CO_2$, cells were collected then and CPM value were measured with liquid scintillation counter, specific inhibition ratio (Pi) was indicated as the activity of natural killer cell, Pi of NLEJ emulsion was compared with the Pi of lentinanin, blank emulsion and the medium were taken as control.

$$Pi = (1-(CPM \text{ value of treatment/CPM value of control})) \times 100\%$$

Results

The results showed that NLEJ stimulated the activity of natural killer cell of mouse in vitro as like as lentinanin did (Table 8). The negative correlation relationship between dose and CPM value can be seen also.

Table 8

| Influence of NLEJ Emulsion on the Activity of Natural Killer Cell of Mouse in vitro | | | |
|---|---|---|---|
| Treatment | Sample (Concentration) | CPM ($\overline{X}\pm$SD) | Pi % |
| NLEJ | 100(1:4) | 5840 $\pm$ 1045** | 52.2 |
| NLEJ | 100(1:2) | 6830 $\pm$ 1085** | 44.1 |
| NLEJ | 100(1:1) | 8355 $\pm$ 1250** | 31.7 |
| NLEJ | 100(mother) | 10925 $\pm$ 790 | 12.7 |
| Lentinanin | 100(1:4) | 5544 $\pm$ 855** | 54.5 |
| Leutinanin | 100(1:2) | 9395 $\pm$ 995** | 35.5 |
| Leutinanin | 100(1:1) | 8130 $\pm$ 930** | 33.5 |
| Leutinanin | 100(mother) | 8625 $\pm$ 1135 | 29.5 |
| Blank | 100(mother) | 12710 $\pm$ 1125 | |
| Control | 100(mother) | 12235 $\pm$ 725 | |

** P<0.01 compared with Blank and Control

(3) Effect of NLEJ Emulsion on Proliferation of Spleen Lymphatic Cell of Mouse Bearing Tumor in vivo

Methods

1 x 10$^4$ leukaemia cells L1210 were inoculated subcutaneously at each mouse's armpit, mice were divided into 6 groups (10 mice/group) at random the next day of inoculating, the 6 treatments were 6.25, 12.5, 25ml/kg of NLEJ, 10ml/kg of lentinanin, blank emulsion and physiological saline, killed the mice after administered 7 times every day, spleen cell suspension obtained aseptically from the mice was regulated to be the cell density of 1 x 10$^7$/ml, 100ul cell suspension and 100ul medium was added into each well, duplicated 3 wells, after incubated at 37°C and under 5% $CO_2$ for 48hr, 0.5uci/well $^3$H-Tdr was added, incubated 20hr more, measured the CPM value of collected cells to compare with the control cells.

Results

It can be seen from Table 9 that NLEJ emulsion could increase the proliferation of spleen lymphatic cell of the mouse bearing leukaemia. The enhancement of NLEJ emulsion on mouse immunity was also increased as the dose increased. The enhancement on mouse immunity was observed in the treatment of lentinanin.

Table 9

| Effect of NLEJ Emulsion on Proliferation of Spleen Lymphatic Cell of Mouse Bearing Tumor in vivo | | |
|---|---|---|
| Treatment | Dose and Administration | CPM ($\overline{X}\pm$SD) |
| Lentinanin | 10ml/kg iv $\times$ 7 | 7410 $\pm$ 770** |
| NLEJ | 6.25ml/kg iv $\times$ 7 | 8970 $\pm$ 415** |
| NLEJ | 12.5ml/kg iv $\times$ 7 | 10720 $\pm$ 565** |
| NLEJ | 25.0ml/kg iv $\times$ 7 | 14330 $\pm$ 360** |
| Blank | 10ml/kg iv $\times$ 7 | 5690 $\pm$ 1180$\Delta$ |
| Control | 10ml/kg iv $\times$ 7 | 5230 $\pm$ 455 |

** P <0.01 compared with blank or control
$\Delta$ P <0.01 compared with control

(4) Effect of NLEJ Emulsion on Activity of NK Cell of Mouse Bearing Leukaemia in vivo

Methods

1 x $10^4$ leukaemia cells L1210 were inoculated subcutaneously at each mouse's armpit, mice were divided into 6 groups (10 mice/group) at random the next day of inoculating, the 6 treatments were 6.25, 12.5, 25ml/kg of NLEJ, 10ml/kg of lentinanin, blank emulsion and physiological saline, killed the mice after administered 7 times every day, spleen cell suspension obtained aseptically from the mice was regulated to be the cell density of 1 x $10^6$/ml, 100ul the cell suspension(effective cell) and 100ul YAC cells(target cell, with the cell density of 1 x $10^4$), and 0.5uci/well 3H-Tdr was added into each well, duplicated 3 wells, after incubated at 37°C and under 5% $CO_2$ for 24hr, measured the CPM value of collected cells, specific inhibition ratio (Pi) was indicated as the activity of natural killer cell.

$$Pi = (1 - (CPM \text{ value of tested group/CPM value of control group})) \times 100\%$$

Results

Table 10 shows the stimulation of NLEJ emulsion and lentinanin to the activity of natural killer cell of mouse bearing leukaemia cells, indicating the promotion of mouse immunity.

Table 10

| Effect of NLEJ Emulsion on Activity of NK Cell of Mouse Bearing Leukaemia in vivo | | | | |
|---|---|---|---|---|
| Treatment | Dose and Administration | | CPM ($\overline{X}\pm$SD) | Pi % |
| Lentinanin | 10ml/kg | iv $\times$ 7 | 5660 $\pm$ 260** | 54.5** |
| NLEJ | 6.25ml/kg | iv $\times$ 7 | 10350 $\pm$ 1600 | 16.8 |
| NLEJ | 12.5ml/kg | iv $\times$ 7 | 8000 $\pm$ 960** | 34.2** |
| NLEJ | 25ml/kg | iv $\times$ 7 | 6210 $\pm$ 890** | 50.1** |
| Blank | 10ml/kg | iv $\times$ 7 | 12510 $\pm$ 430$\Delta$ | |
| Control | 10ml/kg | iv $\times$ 7 | 12450 $\pm$ 340 | |

** P <0.01 compared with blank or control
$\Delta$ P <0.01 compared with control

(5) Influence of NLEJ Emulsion on Induction of Interleukin-2 of Mouse Bearing Leukaemia in vivo

Methods

1 x 10-E4 leukaemia cells L1210 were inoculated subcutaneously at each mouse's armpit, mice were divided into 6 groups (10 mice/group) at random the next day of inoculating, the 6 treatments were 6.25, 12.5, 25ml/kg of NLEJ, 10ml/kg of lentinanin, blank emulsion and physiological saline, killed the mice after administered 7 times every day, spleen cell suspension obtained aseptically from the mice was regulated to be the cell density of $1 \times 10^7$/ml, 2ml the cell suspension containing ConA 5ug/ml, duplicated 3 wells, after incubated at 37°C and under 5% $CO_2$ for 24hr, took the supernatant, measured the activity of interleukin-2(IL-2) with the Interleukin-2 depended cell clone CTLL and the method of 3H-Tdr incorporation.

Results

Table 11 shows that all treatment of NLEJ stimulated the induction of interleukin-2 of the mouse bearing tumor.

Table 11

| Influence of NLEJ Emulsion on Induction of Interleukin-2 of Mouse Bearing Leukaemia in vivo | | |
|---|---|---|
| Treatment | Dose and Administration | | CPM ($\overline{X} \pm$SD) |
| Lentinanin | 10ml/kg | iv $\times$ 7 | 2750 $\pm$ 123** |
| NLEJ | 6.25ml/kg | iv $\times$ 7 | 1460 $\pm$ 184 |
| NLEJ | 12.5ml/kg | iv $\times$ 7 | 2080 $\pm$ 386 |
| NLEJ | 25ml/kg | iv $\times$ 7 | 6020 $\pm$ 910** |
| Blank | 10ml/kg | iv $\times$ 7 | 1750 $\pm$ 487$\Delta\Delta$ |
| Control | 10ml/kg | iv $\times$ 7 | 1830 $\pm$ 95 |

** P <0.01 compared with blank or control
$\Delta\Delta$ P >0.01 compared with control

(6) Influence of NLEJ Emulsion on Phagocytosis Activity of Mouse Phagocyte in vivo

Methods

Swiss mice were divided into two groups at random, one group administered NLEJ emulsion intraperitoneally 7 times every day, the another administered blank emulsion in the same way, 0.5 ml hydrolyzed lactalbumin administered intraperitoneally after last administration, 1 ml erythrocyte suspension (2%) of chicken was given the next day in the same way, 30 min later killed the Swiss mice by the dislocation of cervical vertebra, fix the Swiss mice face up, open the peritoneum, washed the abdominal cavity with 2 ml physiological saline for 1 min, took out 1 ml of the water and drip on the surface of glass, incubated at 37°C for 30 min keeping moisture, washed down the unstuck cells after then, fixed the cells on glass with acetone:methanol 1:1 (v/v) after drying, dyed the cells with 4% Giemsa-phosphate buffer for 3 min, washed with distilled water and dried, counted the phagocyte(more than 100) under microscope, calculated the phagocytosis percentage (PP) and phagocytosis index (PI) as the following:

$$\text{Phagocytosis percentage} = (\text{number of phagocyte phagocytosising erythrocyte / number of the all phagocyte}) \times 100\%$$

$$\text{Phagocytosis index} = (\text{number of erythrocytes being phagocytosised / number of phagocyte}) \times 100\%$$

Results

It can be seen in Table 12 that NLEJ promoted the phagocytosis activity of the mouse phagocyte.

Table 12

| Influence of NLEJ Emulsion on Phagocytosis Activity of Mouse Phagocyte in vivo | | | | |
|---|---|---|---|---|
| Dose/day | PP % | P value | PI % | P value |
| 6.25ml/kg | 31.05 ± 1.96 | <0.01 | 0.68 ± 0.025 | <0.01 |
| 12.5ml/kg | 51.45 ± 3.28 | <0.01 | 1.75 ± 0.14 | <0.01 |
| Control | 14.00 ± 1.08 | - | 0.34 ± 0.024 | - |

**Example 12**

Anti-tumor Activity of NLEJ Emulsion Combined with Small Dose Chemotherapy

(1) Anti-tumor Therapeutical Effect on Walaer Carcinosarcoma (Solid) W256 in vivo

Methods

Ascites Walaer carcinosarcoma (solid) W256 tissues growing luxuriantly were digested to be cell suspension, regulated the cell suspension to be $1-2 \times 10^{7}$/ml cell density, 0.2 ml the suspension was inoculated under the skin of armpit of each rat, the rats were divided into groups at random the next day after inoculating and administered, the doses of NLEJ emulsion were 20ml/kg, 10ml/kg, 5ml/kg iv x 10 (qd), the doses of cyclophosphamide were 30mg/kg iv x 7 (qd), 10mg/kg iv x 2 (third and fifth), tumor tissues of all treatment were sampled and weighted to calculate tumor inhibition ratio on the 14 days after inoculating.

Results

Anti-tumor therapeutical effect of NLEJ emulsion on ascites Walaer carcinosarcoma (solid) W256 was promoted by small dose chemotherapy of cyclophosphamide (Table 13).

Table 13

| Anti-tumor Therapeutical Effect on Walaer Carcinosarcoma (Solid) W256 in vivo | | | | | | |
|---|---|---|---|---|---|---|
| Sample | Dose (ml/kg) | Administration | Number | Weight (g) | Inhibition (%) | P value |
| NLEJ | 20 | iv × 10 | 10/10 | 1.41±0.78 | 58.16 | <0.01 |
| NLEJ | 10 | iv × 10 | 10/10 | 1.83±0.77 | 45.69 | <0.01 |
| NLEJ | 5 | iv × 10 | 10/9 | 2.078±1.1 | 38.34 | <0.05 |
| CTX | 10mg | iv × 2 | 10/10 | 2.07±1.11 | 38.58 | <0.05 |
| CTX | 30mg | iv × 7 | 10/8 | 0.4±0.15 | 88.13 | <0.01 |
| NLEJ+CTX | 20ml+10mg | iv × 2 | 10/10 | 0.93±0.34 | 72.40 | <0.01 |
| NLEJ+CTX | 10ml+10mg | iv × 2 | 10/10 | 1.09±0.5 | 67.66 | <0.01 |
| NLEJ+CTX | 5ml+10mg | iv × 2 | 10/10 | 1.72±0.91 | 48.96 | <0.01 |
| Blank | | iv × 10 | 10/10 | 3.37±1.1 | - | - |

(2) Anti-tumor Therapeutical Effect on Sarcoma (S-180)

Methods

Sarcoma (S-180) tissues growing luxuriantly were prepared to be cell suspension with physical saline 1:3 (w/v), mice were divided into groups at random the next day after inoculating and administered, the dose of NLEJ emulsion was 25 ml/kg iv x 7 (qd), the dose of cyclophosphamide was 30mg/kg ip x 2 (second and fourth), the dose of mitoxantrone (DHAD) was 2mg/kg ip x2 (second and fourth), the dose of mitomycin was 1.5mg/kg ip x2 (second and fourth), tumor tissues of all treatment were sampled and weighted to calculate tumor inhibition ratio on the 14 days after inoculating.

Results

Results showed that anti-tumor therapeutical effect of NLEJ emulsion on sarcoma was prompted by small dose of cyclophosphamide, DHAD and mitomycin (Table 14).

Table 14

| Anti-tumor Therapeutical Effect on Sarcoma (S-180) in vivo | | | | | |
|---|---|---|---|---|---|
| Treatment | Dose and Administration | | No. | Inhibition % | Pi % |
| NLEJ | 25ml/kg | iv × 7 | 10/10 | 23 | <0.05 |
| NLEJ+CTX | 25ml/kg+30mg/kg | ip × 2(1,3) | 10/10 | 44 | <0.01 |
| CTX | 30mg/kg | ip × 2(1,3) | 10/10 | 39.4 | <0.05 |
| NLEJ+DHAD | 25ml/kg+2mg/kg | ip × 2(1,3) | 10/10 | 67 | <0.01 |
| DHAD | 2mg/kg | ip × 2(1,3) | 10/10 | 50 | <0.01 |
| CLEJ+mitomycin | 25ml/kg+1.5m/kg | ip × 2(1,3) | 10/10 | 67 | <0.01 |
| Mitomycin | 1.5mg/kg | ip × 2(1,3) | 10/10 | 39.4 | <0.05 |
| Control | 25mg/kg | ip × 2(1,3) | 30/10 | | |

**Example 13**

Protection of NLEJ Emulsion on Decrease of Leucocyte Induced by Chemotherapy

Methods

80 Swiss mice were divided into eight groups at random, administered with cyclophosphamide 45mg/kg ip x 3 (second, sixth and tenth), harringtonine (HRTN) 1mg/kg ip x 3 (second, sixth and tenth), each two of the medicine groups were administered with NLEJ emulsion the same time with the doses 2.4ml/kg and 6.25 ml/kg iv x 7 (every day), at the beginning and on the fourth day of each administration blood were sampled from the angle of each mouse's eye, leucocyte of the blood were counted.

Results

Table 15 and Table 16 show that NLEJ emulsion can protect mouse from leucocyte decrease induced either from cyclophosphamide chemotherapy or from harringtonine chemotherapy.

Table 15

| Protection of NLEJ Emulsion on Decrease of Leucocyte Induced by Cyclophosphamide Chemotherapy | | | | | | | | | | | | |
|---|---|---|---|---|---|---|---|---|---|---|---|---|
| Medi. | Dose | Administration | No. | Leucocyte number x 100/mm$^3$ ($\overline{X}\pm SD$) | | | | | | | | |
| | | | | 0 | 3 | 6 | 9 | 12 | 15 | 18 | 21 | 24 |
| CTX+ NLEJ | 45mg/kg+ 12.5ml/kg | ip $\times$1,5,9+ iv $\times$7 | 58 ±10 | 62 ±12 | 41 ±6 | 39 ±10 | 43 ±6 | 76** ±11 | 66** ±14 | 61* ±17 | 66** ±12 |
| CTX+ NLEJ | 45mg/kg+ 6.25ml/kg | ip $\times$ 1,5,9+ iv $\times$7 | 65 ±11 | 60 ±9 | 50 ±8 | 39 ±9 | 62 ±9 | 81** ±13 | 69** ±10 | 65** ±10 | 64* ±17 |
| CTX | 45mg/kg | ip $\times$1,5,9 | 62 ±8 | 49$^\Delta$ ±8 | 45$^\Delta$ ±8 | 41$^\Delta$ ±6 | 41$^\Delta$ ±11 | 42$^\Delta$ ±8 | 45$^\Delta$ ±9 | 47$^\Delta$ ±9 | 49$^\Delta$ ±8 |
| Control | | | 58 ±7 | 69 ±2 | 69 ±2 | 68 ±2 | 88 ±3 | 82 ±2 | 81 ±4 | 86 ±2 | 72$^\Delta$ ±2 |

$\Delta$ P<0.001 compared with Control

** and * P<0.01 and <0.05 compared with CTX3,1

Table 16

| Protection of NLEJ Emulsion on Decrease of Leucocyte Induced by Harringtonine Chemotherapy | | | | | | | | | | | | |
|---|---|---|---|---|---|---|---|---|---|---|---|---|
| Medi. | Dose | Administration | Leucocyte number x 100/mm$^3$ ($\overline{X}\pm SD$) | | | | | | | | | |
| | | | 0 | 3 | 6 | 9 | 12 | 15 | 18 | 21 | 24 |
| HRTN+ NLEJ | 1mg/kg+ 12.5ml/kg | ip $\times$1,5,9+ iv $\times$7 | 59 ±8 | 73 ±10 | 61 ±6 | 56 ±11 | 81** ±5 | 89** ±9 | 75** ±16 | 79** ±20 | 97** ±20 |
| HRTN+ NLEJ | 1mg/kg+ 6.25ml/kg | ip $\times$1,5,9+ iv $\times$7 | 65 ±13 | 67 ±4 | 75 ±9 | 70 ±17 | 96** ±18 | 98** ±10 | 89** ±17 | 86** ±22 | 89** ±27 |
| HRTN | 1mg/kg | ip $\times$1,5,9 | 60 ±9 | 75 ±5 | 65 ±10 | 50$^\Delta$ ±10 | 44$^\Delta$ ±10 | 43$^\Delta$ ±7 | 49$^\Delta$ ±8 | 53$^\Delta$ ±9 | 56$^\Delta$ ±9 |
| Control | | | 58 ±7 | 69 ±2 | 69 ±2 | 68 ±2 | 88 ±3 | 82 ±2 | 81 ±4 | 86 ±2 | 72$^\Delta$ ±2 |

$\Delta$ P<0.001 compared with Control

** P<0.01 compared with HRTN

**Example 14**

Clinical Therapeutic Effect of NLEJ Emulsion

Methods

NIEJ Emulsion was administered intravenously or intraarterial in various human cancer therapies. Detail therapeutic methods is omitted. Results

Table 17

| Therapeutic Effect of NLEJ Emulsion | | | | | |
|---|---|---|---|---|---|
| Patient No. | CR | PR | NC | PD | Effective ratio % |
| 51 | 0 | 26 | 19 | 6 | 50.98 |

Table 18

| Improvement of Symptom after Administration | | | | | | |
|---|---|---|---|---|---|---|
| Symptom | No. | Ease | Vanish | Unchange | Worse | Retarded % |
| Pain | 45 | 31 | 10 | 4 | 0 | 91.1 |
| Fever | 29 | 11 | 8 | 7 | 3 | 65.5 |
| Jaded appetite | 84 | 57 | 16 | 10 | 1 | 73.0 |
| Abdominal distension | 38 | 19 | 2 | 12 | 5 | 55.3 |
| Insonomnia | 41 | 21 | 11 | 9 | 0 | 78.0 |
| Powerless | 70 | 37 | 16 | 9 | 8 | 75.7 |
| Cough | 28 | 14 | 6 | 8 | 0 | 71.4 |
| Breath with difficulty | 11 | 5 | 1 | 5 | 0 | 54.5 |
| Irregular Stool | 17 | 3 | 8 | 6 | 8 | 64.7 |

Table 19

| Enhancement of Human Immunity from NLEJ Emulsion | | | | | | |
|---|---|---|---|---|---|---|
| Immunity | Administ. | No. | Before | After | t value | P value |
| ANAE (%) | NLEJ | 40 | $39.38 \pm 7.92$ | $44.46 \pm 13.41$ | 2.477 | <0.05 |
| | Chemotherapy | 19 | $50.05 \pm 7.69$ | $44.79 \pm 6.64$ | 2.580 | <0.05 |
| $OKT_1$ | NLEJ | 40 | $53.47 \pm 6.77$ | $56.83 \pm 7.07$ | 3.670 | <0.001 |
| | Chemotherapy | 19 | $67.45 \pm 4.60$ | $62.91 \pm 6.46$ | 2.424 | <0.05 |
| $OKT_4/OKT_8$ | NLEJ | 40 | $1.09 \pm 0.20$ | $1.16 \pm 0.33$ | 2.098 | <0.05 |
| | Chemotherapy | 19 | $1.39 \pm 0.25$ | $1.26 \pm 0.28$ | 2.424 | <0.05 |
| NK Cell (%) | NLEJ | 40 | $34.82 \pm 12.41$ | $43.91 \pm 16.97$ | 3.222 | <0.01 |

Table 20

| Protection of Marrow Function from NLEJ Emulsion | | | | | |
|---|---|---|---|---|---|
| Value | Therapy | No. | HB | WBC | PC |
| Increase | After | 102 | 80 | 85 | 79 |

Table 20 (continued)

| Protection of Marrow Function from NLEJ Emulsion | | | | | |
|---|---|---|---|---|---|
| Value | Therapy | No. | HB | WBC | PC |
| Unchange | After | 102 | 2 | 2 | 4 |
| Decrease | After | 102 | 20 | 15 | 19 |
| Effective% | | | 78.43 | 83.33 | 77.45 |
| Average | Before | 102 | 10.35±1.77 | 4.82±1.47 | 9.31±4.27 |
| | After | 102 | 10.64±1.96 | 5.78±1.70 | 10.70±4.51 |
| t value | | | 2.343 | 6.302 | 5.070 |
| P value | | | <0.05 | <0.001 | <0.001 |

Table 21

| Improvement of Function of Liver and Spleen from NLEJ | | | | | | | |
|---|---|---|---|---|---|---|---|
| Index | No. | Before | | After | | | Retarded(%) |
| | | Normal | Abnormal | Normal | Retard | Unchange | |
| Bilirubin | 70 | 52 | 10 | 52±4 | 6 | 8 | 55.56 |
| G.P.T. | 70 | 58 | 12 | 58±4 | 5 | 3 | 75.00 |

Table 22. Strength Improvement from NLEJ Emulsion

| Patient No. | K P S Evaluation | | | | | Increasing % |
|---|---|---|---|---|---|---|
| | +30 | +20 | +10 | Stable | decrease | |
| 70 | 16 | 22 | 15 | 9 | 8 | 75.71 |

| Therapy | No. | K P S Evaluation | | | $X^2$ value | P value |
|---|---|---|---|---|---|---|
| | | >70 | 50-60 | <50 | | |
| Before | 70 | 15 | 28 | 27 | | |
| After | 70 | 39 | 18 | 13 | 21.159 | <0.001 |

| | KPS Evaluation | NLEJ (n=51) | Chemotherapy (n=49) | $X^2$ value | P value |
|---|---|---|---|---|---|
| Before | >70 | 13 | 40 | | |
| | 50-60 | 18 | 7 | | |
| | <50 | 20 | 2 | 29.904 | <0.001 |
| After | >70 | 31 | 20 | | |
| | 50-60 | 10 | 21 | | |
| | <50 | 10 | 8 | 6.459 | <0.05 |
| | $X^2$ value | 12.934 | 17.266 | | |
| | P value | <0.001 | <0.001 | | |

## Claims

1. Neutral lipids from the endosperm of Job's tears (Coix lachryma - jobi) (NLEJ), having the following physicochemical indexes: acid value <0.20, iodine value 95.00-107.00, saponification value 185.00-195.00, relative density 0.915-0.918 (20°C) and diopter 1.470-1.475 (20°C).

2. NLEJ according to claim 1 consisting essentially of glycerides wherein the lipoclastic fatty acid residue of said NLEJ comprise alkyl fatty acids and alkenyl fatty acids.

3. NLEJ of claim 2 wherein said glycerides comprise triglyceride and monoglyceride.

4. NLEJ of claim 2 or claim 3 wherein said lipoclastic fatty acid residues of NLEJ comprise essentially hexadecanoic, octodencanoic, octadecenoic and octadecadienoic acid.

5. A method for the preparation of NLEJ according to claim 1, comprises the steps of :

   (a) extraction of dry endosperm of Job's tears by organic solvent, absorption by absorbent and decoloration;
   (b) alkalizing saponification;
   (c) demulsification by acetone; and
   (d) extraction by an organic solvent.

6. The method of claim 5 wherein said organic solvent being used in step (a) is acetone.

7. The method of claim 5 wherein the organic solvent of step (d) is petrol ether.

8. The method of claim 5, claim 6 or claim 7 wherein step (b) comprise the use of NaOH or KOH.

9. The method of any one of claims 5 to 8 which comprises also the following steps:

   (1) the step of washing the emulsion with hot water between alkalinity saponification and demulsification; and/or
   (2) the step of absorption and decoloration added after the extraction of NLEJ by organic solvent; and/or
   (3) the step of washing by hot water added after the extraction of NLEJ by organic solvent.

10. A pharmaceutical composition which comprises NLEJ, according to claim 1.

11. The pharmaceutical composition of claim 10 which is an anti-tumor oil in water emulsion, said composition also including emulsifier and isoosmoticum.

12. The pharmaceutical composition of claim 11 wherein said composition is :

| NLEJ | 5-25g |
|---|---|
| Soy or egg lecithin | 0.3-3.0g |
| Glycerol | 1.25-6g |
| Distilled water | q.s.to 100ml |

13. The pharmaceutical composition of claim 12 wherein said composition is:

| NLEJ | 10g |
|---|---|
| Soy lecithin for injection use | 1.5g |
| Glycerol for injection use | 2.5g |
| Water for injection use | q.s. to 100ml |

14. The pharmaceutical composition of any one of claims 10-13 which comprises other anti-tumor drugs.

15. NLEJ according to claim 1 for use as a medicament.

**Patentansprüche**

1. Neutrale Lipide aus dem Endosperm von Hiobstränen (Coix lachryma jobi) (NLEJ) mit den folgenden physikochemischen Indizes: Säurezahl < 0,20, lodzahl = 95,00 - 107,00, Verseifungszahl = 185,00 - 195,00, relative Dichte = 0,915 - 0,918 (20 °C) und Dioptrie = 1,470 - 1,475 (20 °C).

2. NLEJ nach Anspruch 1, die im wesentlichen aus Glyceriden bestehen, worin die lipolytischen Fettsäurereste der NLEJ Alkylfettsäuren und Alkenylfettsäuren umfassen.

3. NLEJ nach Anspruch 2, worin die Glyceride Triglyceride und Monogylceride umfassen.

4. NLEJ nach Anspruch 2 oder 3, worin die lipolytischen Fettsäurereste der NLEJ im wesentlichen Hexadecan-, Octodecan-, Octadecen- und Octadecadiensäure umfassen.

5. Verfahren zur Präparation von NLEJ nach Anspruch 1, folgende Schritte umfassend:

   (a) die Extraktion von trockenem Endosperm von Hiobstränen mit organischem Lösungsmittel, Absorption mit Absorbens und Entfärbung;
   (b) alkalisierende Verseifung;
   (c) Demulgierung mit Aceton; und

(d) Extraktion mit einem organischen Lösungsmittel.

6. Verfahren nach Anspruch 5, worin das in Schritt (a) verwendete organische Lösungsmittel Aceton ist.

7. Verfahren nach Anspruch 5, worin das organische Lösungsmittel in Schritt (d) Petrolether ist.

8. Verfahren nach Anspruch 5, 6 oder 7, worin Schritt (b) die Verwendung von NaOH oder KOH umfaßt.

9. Verfahren nach einem der Ansprüche 5 bis 8, das auch die folgenden Schritte umfaßt:

(1) den Schritt des Waschens der Emulsion mit heißem Wasser zwischen der alkalisierenden Verseifung und der Demulgierung; und/oder
(2) den zusätzlichen Schritt der Absorption und Entfärbung nach der Extraktion der NLEJ mit organischem Lösungsmittel; und/oder
(3) den zusätzlichen Schritt des Waschens mit heißem Wasser nach der Extraktion der NLEJ mit organischem Lösungsmittel.

10. Pharmazeutische Zusammensetzung, die NLEJ nach Anspruch 1 umfaßt.

11. Pharmazeutische Zusammensetzung nach Anspruch 10, die eine Anti-Tumor-Öl-in-Wasser-Emulsion ist, wobei die Zusammensetzung auch Emulgator und Isoosmotikum umfaßt.

12. Pharmazeutische Zusammensetzung nach Anspruch 11, worin die Zusammensetzung besteht aus:

| NLEJ | 5 - 25 g |
|---|---|
| Soja- oder Ei-Lecithin | 0,3 - 3,0 g |
| Glycerin | 1,25 - 6 g |
| Destilliertes Wasser | auf 100 ml. |

13. Pharmazeutische Zusammensetzung nach Anspruch 12, worin die Zusammensetzung besteht aus:

| NLEJ | 10 g |
|---|---|
| Soja-Lecithin zur Injektion | 1,5 g |
| Glycerin zur Injektion | 2,5 g |
| Wasser zur Injektion | auf 100 ml. |

14. Pharmazeutische Zusammensetzung nach einem der Ansprüche 10 bis 13, die andere Anti-Tumor-Arzneimittel umfaßt.

15. NLEJ nach Anspruch 1 zur Verwendung als Medikament.

**Revendications**

1. Lipides neutres de l'endosperme des larmes de Job (<u>Coix lachryma - jobil</u>) (NLEJ), ayant les indices physicochimiques suivants: valeur d'acide <0,20, valeur d'iode 95,00-107,00, valeur de saponification 185,00-195,00, densité relative 0,915-0,918 (20°C) et dioptrie 1,470-1,475 (20°C).

2. NLEJ selon la revendication 1 consistant essentiellement en glycérides où le résidu d'acide gras lipoclastique dudit NLEJ comprend des acides alkyles gras et des acides alcényles gras.

3. NLEJ de la revendication 2 où lesdits glycérides comprennent le triglycéride et le monoglycéride.

4. NLEJ de la revendication 2 ou de la revendication 3 où lesdits résidus d'acide gras lipoclastique de NLEJ comprennent essentiellement l'acide hexadécanoïque, octodécanoïque, octadécénoïque et octadécadiénoïque.

5. Méthode pour la préparation de NLEJ selon la revendication 1, qui comprend les étapes de:

   (a) extraction de l'endosperme sec des larmes de Job par un solvant organique, absorption par un absorbant et décoloration;
   (b) saponification alcalisante;
   (c) démulsification par l'acétone; et
   (d) extraction par un solvant organique.

6. Méthode de la revendication 5 où ledit solvant organique utilisé à l'étape (a) est l'acétone.

7. Méthode de la revendication 5 où le solvant organique de l'étape (d) est de l'éther de pétrole.

8. Méthode de la revendication 5, de la revendication 6 ou de la revendication 7 où l'étape (b) comprend l'utilisation de NaOH ou KOH.

9. Méthode selon l'une des revendications 5 à 8 qui comprend également les étapes suivantes:

   (1) l'étape de laver l'émulsion avec de l'eau chaude entre la saponification à l'alcalinité et la démulsification; et/ou
   (2) l'étape d'absorption et de décoloration ajoutée après l'extraction de NLEJ par un solvant organique; et/ou
   (3) l'étape de laver par de l'eau chaude ajoutée après l'extraction de NLEJ par le solvant organique.

10. Composition pharmaceutique qui comprend NLEJ selon la revendication 1.

11. Composition pharmaceutique de la revendication 10 qui est une émulsion anti-tumeur huile dans l'eau, ladite composition comprenant également un agent émulsionnant et de l'isoosmoticum.

12. Composition pharmaceutique de la revendication 11, où ladite composition est:

| NLEJ | 5-25 g |
|---|---|
| Lécithine de soja ou d'oeuf | 0,3-3,0 g |
| Glycérol | 1,25-6 g |
| Eau distillée | s.q. à 100 ml |

13. Composition pharmaceutique de la revendication 12 où ladite composition est:

| NLEJ | 10 g |
|---|---|
| Lécithine de soja pour usage par injection | 1,5 g |
| Glycérol pour usage par injection | 2,5 g |
| Eau pour usage par injection | s.q. à 100 ml |

14. Composition pharmaceutique selon l'une des revendications 10-13 qui comprend d'autres médicaments anti-tumeur.

15. NLEJ selon la revendication 1 pour une utilisation comme médicament.

Fig. 1

Fig. 2

Fig. 3

Fig. 4

Inhibition of Metastasis (%)

Fig. 5